# EUROPEAN PATENT APPLICATION

(11) **EP 3 459 480 A1**
(43) Date of publication of application: **27.03.2019**
(21) Application number: 17192463.2
(22) Date of filing: 21.09.2017
(51) Int. Cl.: A61B 18/14, A61B 18/10

(54) **APPARATUS FOR LOCALISING AN ELECTRICAL FIELD**

(71) Applicant: National University of Ireland, Galway, H91 TK33 Galway (IE)
(72) Inventor: O'Brien, Barry, Galway (IE); Bruzzi, Mark, Salthill Galway (IE); Rabbette, Tadhg, Claregalway, Co. Galway (IE)
(74) Representative: Murgitroyd & Company

(57) **Abstract**

Apparatus for localising an electrical field for electroporation of tissue includes a pulsed DC electrical power supply and at least one catheter tip and electrode assembly configured for endocardial placement such that electrodes positionable at separate endocardial locations allow development of an electrical field between the electrodes to effect electroporation of tissue in the electrical field.

## Description

This disclosure relates to apparatus useful in the medical field. The apparatus may be useful in the treatment of atrial fibrillation.

### Background

Cardiovascular disease is common in the western world and a great deal of research has been carried out in the field to treat and diagnose the various conditions associated with cardiovascular problems. Atrial fibrillation is one heart condition in which the heart beats in an irregular fashion, often much faster than is considered normal. Undiagnosed or untreated atrial fibrillation may lead to weakening of the heart and the potential for heart failure.

Atrial fibrillation can be controlled in suitable subjects by medication or by electrical stimuli (controlled electric shock) to restore a normal rhythm, or by means of catheter ablation of abnormal heart tissue.

According to a known catheter ablation technique, a thin catheter bearing electrodes is introduced to the heart via a blood vessel, and an area of tissue abnormality can be identified according to evaluation of electrical activity of the heart tissue. The area of tissue abnormality can be ablated by introducing high radio frequency energy via the catheter to create localised heat to target the tissue to be ablated. As an alternative to use of heat, a cryoablation technique can be used. Cryoablation may use argon or helium delivered under high pressure via a catheter to achieve temperatures of -55 to -90 °C at the catheter tip. Tissue adjacent the tip may be cooled for about 2 minutes leading to subsequent formation of very localised lesions with adjacent tissue being unaffected. Ablation of the abnormal tissue may allow the restoration of normal rhythm of the heart.

It has been assessed that the known catheter ablation techniques may have a success rate of about 50-60%. Therefore, an alternative approach which may improve on that rate and be suitable for patients with either paroxysmal or persistent atrial fibrillation would be useful. It has also been recognised that the aforesaid radiofrequency or cryoablation techniques may introduce thermal damage to the myocardium which damage offsets benefits of the technique and impairs outcomes for the patients.

Currently catheter ablation using radiofrequency or cryoablation for atrial fibrillation is mostly performed on the endocardial (internal) surfaces, but collateral damage to healthy tissue may induce other arrhythmias such as atrial flutter and atrial tachycardia.

Epicardial (external surface) sites may also contribute to initiation of atrial fibrillation. Sites where clusters of autonomic neurons interface with each other and with the heart itself, are known as the ganglionated plexi sites, which are typically embedded within fat pads on the epicardial surface of the heart, but can be in direct contact with the myocardium. The ganglionated plexi can be ablated using radiofrequency or cryoablation during open surgery, thorascopic surgery, or from an endocardial position via the myocardium. However, such ablation techniques are not well suited to ablation of the ganglionated plexi sites within the transverse sinus and aortocaval sinus (superior sinus) due to the risk of thermal or physical damage to collateral structures.

Work by Lu et al, Journal of Cardiovascular Electrophysiology, 21(12), 1392-1399 (2010), and by Lo et al, Heart Rhythm Volume 10, Issue 5, Pages 751-757 (2013), has shown how the aortocaval ganglionated plexi initiated atrial fibrillation via the vena cava, and indicates that this ganglionated plexi may be the most important due to connection to other ganglionated plexi sites via nerve fibres.

Work by Wang et al, Journal of the American College of Cardiology, EP 2015; 1, : 390-397 has indicated that the aortic root ganglionated plexi has fibre connections to the left pulmonary veins, contributing to atrial fibrillation firing from these. Since the aortic root ganglionated plexi is a ventricular ganglionated plexi, the traditional view would have been that ventricular ganglionated plexi would not influence atrial fibrillation.

Although the aortocaval ganglionated plexi and the aortic root ganglionated plexi are evidently significant with regard to atrial fibrillation in comparison with other ganglionated plexi sites, they are difficult to access surgically, and their proximity to critical arterial structures that could be easily damaged is a relevant factor in devising a procedure. Surgical access to these sites at the top of the heart requires more extensive tissue cutting and displacement compared to other ganglionated plexi on the rear surfaces of the heart. This necessitates longer procedures with the associated risks of longer cardiopulmonary pump time.

In addition to these access challenges, the current energy sources used for ablation (radiofrequency and cryo energy) present a hazard to the arteries and veins that are grouped tightly in this location, including the pulmonary artery, the aorta, the right and left coronary arteries and the superior vena cava. Damage due to RF or cryo energy can cause lesions leading to thrombus, restenosis, and occlusion as well as potentially causing perforation. Taking these challenging factors into consideration, the ganglionated plexi at these sites are not usually targeted which limits the ability to reduce atrial fibrillation through ablation of these autonomic nerve sites.

EP 2 986 243 A1 discloses a method and a device for modulating the autonomic nervous system adjacent a pericardial space to treat cardiac arrhythmia. The device performs modulation or ablation of the autonomic nervous system at selected treatment areas within the pericardium.

### Summary

Electroporation may be considered as an alternative to ablation. This is an athermal process that involves application of high voltage direct current pulses for very short durations which avoids heat build-up, for example applying 1000 volts for 100 µs. However, the efficacy of electroporation is also temperature dependent, with the threshold for electroporation being decreased at elevated temperatures. Electroporation could selectively ablate neurons in a targeted manner without discernible collateral damage to surrounding structures such as myocardium and arterial or venous tissue.

Electrophysiology apparatus disclosed herein comprises a high voltage pulsed DC supply, and at least one catheter configured for endocardial access, the catheter including a catheter tip and electrode assembly connectable with the high voltage pulsed DC supply. The electrophysiology apparatus may comprise a catheter assembly including more than one catheter including a catheter tip and electrode assembly so that the electrophysiology apparatus comprises at least two electroporation electrodes connectable with the high voltage pulsed DC supply such that the at least two electroporation electrodes are oppositely charged.

In this disclosure, a catheter assembly means a plurality of catheters capable of being introduced to target tissue, and arranged to be used together in an operational space including target tissue for the purposes of modifying the target tissue by electroporation. Each such catheter of the catheter assembly may have a proximal end adapted for a user control, for example to manipulate the catheter, and a distal end referred to herein as a catheter tip, which catheter tip is configured for introduction endocardially via a lumen of a natural vessel selected from any of the superior vena cava, the aorta, pulmonary arteries, coronary arteries. The catheter tip may have one of more selectively controllable electrodes located thereon. The catheter tip and electrode(s) together provide a catheter tip and electrode assembly. Each catheter tip and electrode assembly can be used as an electroporation electrode when connected to a high voltage pulsed direct current supply.

In embodiments a single catheter may have multiple catheter tip and electrode assemblies, for example a catheter may have a bifurcated tip having selectively controllable electrodes on each limb of the bifurcated tip.

A suitable catheter may have an internal lumen (throughbore) for passage of electrical conductors or fluids.

In illustrative embodiments the catheter tip and electrode assembly comprises at least three electroporation electrodes provided on separate catheters and connectable with the high voltage pulsed DC supply such that, selectively, at least two of the electroporation electrodes are oppositely charged. In this arrangement wherein multiple electrodes are connectable to the high voltage pulsed DC supply, and selectively switchable such that at least two of the electroporation electrodes are oppositely charged at any one time so as to be capable of causing electroporation to tissue contacted by the electroporation electrodes, and whereby the electric field focus varies but the target tissue to be ablated is continually ablated by means of the different selected pairs of electroporation electrodes according to the selective switching steps.

The catheter tip and electrode assembly may comprise a plurality of catheter tips and each catheter tip may be provided with at least one electrode capable of serving as one of the said at least two electroporation electrodes.

In illustrative embodiments a catheter may have a straight linear tip, or may have multiple tips such as for example a bifurcated tip, or may have a curvilinear tip for example of part circular, or helical form. The respective tips may have spaced thereon a plurality of electrodes, for example from 2 to 8, or a multiplicity of spaced electrodes, say up to 40 or more. In other embodiments, the electrodes are located on an expandable support such as a balloon, or upon a cage or basket support frame. Each electrode may be individually controlled, such that one or more electrodes may be selectively operated alone, in selected coupling combinations, or together at the same time. In such embodiments, differences in patient anatomy or size differences in the target or surrounding tissue can be accommodated.

The apparatus can be configured such that in use all of the electrodes on a single catheter tip have the same polarity and may be operated simultaneously. The apparatus may also be configured such that individual electrodes can be operated independently in order that voltage levels and polarity can be selectively controlled.

Electrodes may be designed to minimise sharp points or edges and preferably have smooth contours so that changes in surfaces, edges, ends or corners are rounded in order to reduce the risk of point concentration of the electric field.

An electrode for mounting upon a catheter tip may be of tubular form, and may be a short hollow cylinder having an outside diameter which may be from 2 to 4 mm but can lie in the range of from 1 mm up to 8 mm, and having a length of from 2 to 10 mm but can range from 0.5 mm up to 20 mm.

An electrode for mounting upon a catheter tip may be of tubular form and have one or more irrigation holes in the cylindrical sidewall allowing transmission of fluid supplied via the catheter to the external surface of the electrode. Such irrigation holes may be used to direct conductive saline towards target tissue, thereby increasing the distance over which the electric field will develop. Unlike conventional electrodes, such irrigation is not required for cooling of the electrode. Thus in alternative embodiments the irrigation hole(s), could be positioned in the surface of the catheter adjacent to the electrode, rather than being within the sidewall of the electrode.

Electrodes may be partially insulated to preferentially direct the electric field in a desired direction, and reduce the effect of the electric field in other directions.

In embodiments, an expandable balloon may be use to provide the desired electrical insulation when appropriately positioned to cover parts of the electrode, leaving selected surface(s) exposed. Use of a balloon may also serve to move the electrode(s) closer to a tissue surface to be treated.

The catheter tip and electrode assembly may comprise a sheath for electrical field blocking. The sheath is designed to prevent electrical fields from being absorbed by collateral structures near to the target tissue. The sheath may be configured to fit over the catheter tip and electrode assembly and may be fenestrated so as o have a plurality of openings for selective electric field emission. The openings serve as "windows" which may expose one or more electrodes to allow selective directional control for the electrical field emissions. The sheath may be adjusted circumferentially or longitudinally to influence electrical field emission.

The sheath may be fenestrated by provision of multiple holes or apertures positioned along the length and around the circumference of the sheath. It will be understood that rotational or axial movement of the sheath can provide for directing the field to different locations.

The sheath may comprise a metal reinforced polymer, such as a metal-braided polymer tubes of a type already widely used in the field for catheter structures. US 6 635 047 B2, as an example, discloses a metal braid reinforced polymer tube for use as a coronary guide catheter. The parts of the tube containing the metal braid structure acts as a Faraday cage such that an electrical field does not pass through it.

The catheter may be referred to as having a proximal portion and a distal portion, wherein "proximal" and "distal" are defined in this disclosure with reference to a user of apparatus, such that the proximal portion may comprise a handle for manipulation of the catheter, and the distal portion may comprise the catheter tip and electrode assembly.

The catheter tip and electrode assembly may be sufficiently flexible to allow spaced positioning of one electrode with respect to another electrode when presented to a surface such as tissue to be treated, and to allow selective orientation of the respective electrodes to alter direction of an electrical field emanating therefrom.

The catheter tip and electrode assembly may incorporate at least one articulation point at a location proximal and adjacent to the electrodes. This allows for deflection of the electrodes such that the orientation of the electrical field lines can be altered. Thus without changing the nominal position of the catheter, a field orientation change can be achieved.

The catheter tip and electrode assembly may be introduced on a common catheter configured for endocardial access, and having deflectable catheter tips whereby electrodes provided on the respective deflectable catheter tips may be individually re-oriented to change electrical field orientation emanating from each electrode.

In other illustrative embodiments, a plurality of catheters may be used to introduce the electrodes, such that for example one catheter may be used to introduce an electrode connectable to the positive terminal of a high voltage pulsed DC supply, and another catheter may be used to introduce an electrode connectable to the negative terminal of the high voltage pulsed DC supply.

The disclosed apparatus is useful for physiological modification of tissue, and can be operated by introduction of the selected catheter(s) to a surgical site , and control of an electrical power source to deliver powerful direct current pulses of short duration whereby a high voltage is applicable to tissue without the drawback of heat associated with electric current. This procedure is known as "Electroporation" and modifies tissue such that the tissue is initially rendered more permeable and at increased energy levels (higher voltages and longer pulse durations) the electroporation can be controlled to cause cell death and thereby achieve an effect equivalent to tissue ablation.
Electroporation of target tissue is achievable using the apparatus disclosed herein, in that when electrodes of opposite charge are brought into contact with tissue a potential difference is applied across the tissue, and that electrode contact completes a circuit, which circuit includes the high voltage pulsed DC supply, electrodes and the tissue, and thereby accomplishes the electroporation of target tissue with no significant or enduring damage to tissue adjacent to the target tissue

Accordingly, according to an aspect there is provided a system for carrying out electroporation of tissue comprising a high voltage pulsed DC supply, and a catheter configured for endocardial access, the catheter including a plurality of catheter tip and electrode assemblies connectable with the high voltage pulsed DC supply such that at least one electrode is positive (+) and a different electrode is negative (-), and a controller operably connected to the high voltage pulsed DC supply, and the plurality of catheter tip and electrode assemblies for selectively controlling the pairing of (+) and (-) electrodes of the catheter tip and electrode assemblies according to a switching sequence, and the duration of an electrical field pulse delivered by the high voltage pulsed DC supply via said paired electrodes, wherein the system is operable to provide an electrical field pulse of a voltage and duration to cause electroporation of target tissue with no significant or enduring damage to tissue adjacent to the target tissue.

According to an aspect, ganglionated plexi can be targeted by a localised electrical field generated around and between catheter tip and electrode assemblies as disclosed herein. Ganglionated plexi contain neurons which may be preferentially susceptible to electroporation in comparison to the myocardium. This susceptibility may be attributable to relative size difference between the two cell types; cardiac myocytes are typically 10 -20 µm in diameter, and 50-100 µm in length whilst a neuron soma (body) can be from 4 to 100 µm in diameter, with the axons extending over 1000 µm in length. Control of the electrical field strength and extending the duration of pulses can shift the electroporation mechanism from reversible to irreversible electroporation. Taking account of the preferential susceptibility of neurons, appropriate control of electrical field strength and the duration of pulses applied to such differing cell types is found to result in selective irreversible electroporation of ganglionated plexi, with the adjacent cardiac myocytes being primarily reversibly electroporated. Since the ganglia are generally to be found embedded in fat pads a pre-heating step is proposed hereinbelow to render the ganglia even more susceptible to electroporation.

In a surgical procedure to counter atrial fibrillation in a patient assessed as capable of benefiting from endocardial electroporation, a known endovascular procedure using fluoroscopic guidance may be adopted initially, which procedure may include use of a Seldinger type technique using an access sheath, guidewire, and guiding catheter, via a femoral vein (left or right), up through the iliac vein, the inferior vena cava, through the right atrium, and into the superior vena cava target site. An electroporation catheter may then be tracked to this site, using the radiopaque electrodes to assist with guidance and positioning.

In a non-limiting example an endocardial target location, say the superior vena cava, may be accessed by femoral vein needle puncture with tracking under fluoroscopic guidance with the patient under general anaesthesia. Alternatively, the superior vena cava can also be accessed via the internal jugular vein, or subclavian vein, which are less common routes, but may be useful in certain circumstances, for example where anatomical variability may make the inferior route more difficult to track, or for example if the patient had a filter within the inferior vena cava.

Access to the right pulmonary artery may also be achievable via the right atrium (inferior or superior route), across the tricuspid valve to the right ventricle and then through the pulmonary valve into the pulmonary trunk. Guidewires and guide catheters can again be used to assist with tracking the electroporation catheter into the right pulmonary artery. Given that this route crosses through the tricuspid and pulmonary valve, the ablation catheter selected would ideally have a profile no larger than 4-5 Fr.

Access to the aorta may be achieved via a standard femoral artery puncture, with the pathway being via the iliac artery to the aorta. The electroporation catheter may be tracked around the aortic arch with the distal end electroporation electrodes positioned proximal to the aortic valve. Conventional needles, access sheaths, guidewires and guide catheters can again be used to assist with access and tracking. This access route may be also used when the ablation catheter needs to be placed in the right or left coronary arteries. When positioning in the proximal coronary arteries the distal end profile of the ablation catheter would also ideally be no larger than 4-5 Fr.

In an embodiment of the electroporation aspect of the procedure, a first catheter tip and electrode assembly may be introduced by a selected pathway, and a second catheter tip and electrode assembly may be introduced via an alternative pathway, so that the first and second catheter tip and electrode assemblies form a pair of electrodes between which an electrical field may be generated to allow electroporation of target tissue and achieve a tissue modulation equivalent to ablation by any of the prior art methods, but with reduced collateral tissue damage.

Upon completion of the desired electroporation procedure, the electroporation catheter would be withdrawn first, followed by removal of the guide catheter and access sheath. A standard vascular closure technique can then be used to achieve haemostasis at the access site.

As an optional method for periprocedural measurement of electroporation success, the atrial refractory period may be measured before and after electroporation. Extension of the atrial refractory period is an indicator that atrial fibrillation is less likely to be induced and sustained. These measurements can be done using standard electrophysiology devices and techniques and can also be used to get a measure of atrial fibrillation inducibility. An example of such a pacing protocol is described by RB Krol et al (Journal of Interventional Cardiac Electrophysiology 1999; 3: 19-25). Another potential measurement tool would be 1-123-Metaiodobenzylguanidine (MIBG) imaging to assess innervation before and after ganglia ablation. R Lemery et al (Heart Rhythm 2017; 14(1): 128-132) describe this method.

The following documents which mention electroporation may be useful in understanding the background to this disclosure: US2002/0040204, and US2016/0051324.

It will be understood by those in the field that the efficacy of electroporation depends on the orientation of the cell axis relative to the electrical field (Tung et al Circulation Research 1991) with maximum effect when the cell axis is parallel to the field axis. As the orientation of neuronal cell structures within a ganglion is likely to be somewhat random, this ability to shift the electrical field orientation will provide more effective electroporation. The articulation can be provided by any appropriate method, such as for example, pull wires that can be manipulated proximally to give the distal motions. The articulation can also be used simply as an additional adjustment to achieve the best nominal position of the electrode relative to the target anatomy and the coupling electrode(s).

The catheter tip and electrode assembly may be configured to be positioned and repositionable within an operational space, for example a spherical volume of space.

The operational space may surround target tissue within or adjacent to a natural vessel lumen or natural organ cavity. The natural vessel may be any of the superior vena cava, the aorta, pulmonary arteries, coronary arteries, for example and the organ may be the heart, and the cavity may be the atrium or ventricle chambers of the heart. The target tissue may be any of the ganglionated plexi adjacent to or accessible via the said natural vessels.

In the electroporation of cells and tissue using electrophysiology apparatus and methods as disclosed herein at least two electrodes of opposite charge may be located at separate endocardial locations such that an electrical field can be developed between these electrodes. An advantage of this approach is that epicardial structures such as the ganglionated plexi in the transverse sinus can be ablated without needing to gain epicardial access.

Optionally, the target ganglionated plexi may be pre-heated by raising the temperature of the surrounding fat pads using targeted laser energy, or introducing localised warm saline which could be flushed directly into the target area, for example into the pericardial space, particularly over the transverse sinus and oblique sinus areas, or a warm saline-containing balloon could be positioned at discreet ganglia locations. Such a balloon may be introduced as a separate epicardial device or integrated into an epicardial electroporation catheter for the electroporation apparatus. A suitable laser may be of the NdYAG or Diode type. A tunable laser may be used, or the gain adjusted to select a wavelength which will preferentially target fat as opposed to heating surrounding tissue. Optionally ultrasound may be used to heat the fat pads surrounding the ganglionated plexi.

Optionally, heating of the fat pads could be complemented by cooling the myocardium, for example by introducing a chilled saline balloon, whereby the temperature of the myocardium may be cooled locally to about 5-30°C.

Alternatively, cooling of the myocardium may be conducted in the absence of heating of the fat pads. Optionally, cooling may be effected by cooling of the blood in the pulmonary veins through provision of a catheter adapted to cool the blood as it flows past the catheter.. Use of a cooling catheter and/or a cooling balloon, optionally in combination, in the proximity of the pulmonary veins may be employed.

The optional heating and cooling steps disclosed herein may be also used in conjunction with endocardial access procedures.

The disclosed methods and apparatus will now be further described with reference to the accompanying drawings in which:
Fig. 1 illustrates use of a loop shaped catheter tip with multiple electrodes positioned upon the loop shaped tip, wherein one such catheter (+) is positioned in the aorta, and the second (-) is positioned in the superior vena cava;
Fig. 2 illustrates use of several loop shaped catheter tips with multiple electrodes positioned upon the loop shaped tip, wherein one such catheter tip is positioned in the aorta, another catheter tip is positioned in the superior vena cava, and a further catheter tip is positioned in the right pulmonary artery.
Fig. 3a to 3c illustrates schematically use of three catheter mounted electrodes arranged in proximity to the ganglionated plexi and operated as opposed charge pairs in a progressive electroporation sequence for effecting ablation of tissue;
Fig. 4 illustrates schematically use of a three electrodes (+),(+),(-) assembly in a higher field density ablation treatment;
Fig. 5 illustrates schematically use of a three electrodes (+),(+),(-) assembly in a variable electrical field geometry ablation treatment;
Fig. 6 illustrates examples of selected sites for positioning of catheter mounted electrodes for electroporation of the aortic root ganglionated plexi;
Fig. 7 illustrates schematically simultaneous heating of a fat pad including ganglia, with cooling of the myocardium;
Fig. 8 illustrates schematically electroporation of the heated fat pad and ganglia after heating and cooling as illustrated in Fig. 7;
Fig. 9 illustrates schematically a sheath wherein there is a circular aperture;
Fig. 10 illustrates schematically a sheath wherein there is rectangular aperture;
Fig. 11 represents a partially cutaway view of a braided sheath;
Fig. 12 illustrates schematically a catheter tip and electrode assembly positioned within an apertured sheath exposing electrical field emission from an electrode ;
Fig. 13 illustrates schematically a straight linear catheter tip and electrode assembly;
Fig. 14 illustrates schematically a bifurcated linear catheter tip and electrode assembly;
Fig. 15 illustrates schematically a curvilinear catheter tip and electrode assembly.

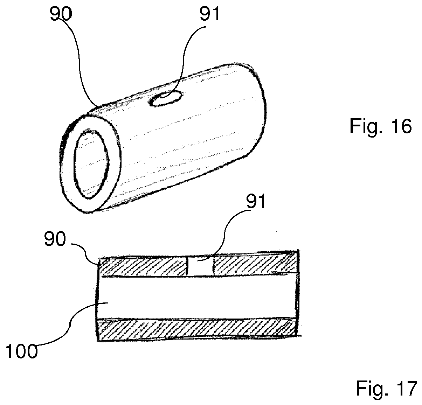

Fig. 16 illustrates schematically a portion of a tubular electrode to be fitted over a catheter and showing an optional irrigation hole for transmission of fluid;
Fig. 17 illustrates a longitudinal section through the portion of a tubular electrode illustrated in Fig, 16
Fig. 18 illustrates schematically a portion of a catheter tip and electrode assembly which is partially insulated, optionally with an inflatable envelope or "balloon"; and
Fig. 19 shows a transverse section across the portion of a catheter tip and electrode assembly illustrated in Fig. 18.

### Example 1

### Apparatus for use in treating the aortocaval ganglionated plexi

In an embodiment intended for treating the aortocaval ganglionated plexi using at least two electroporation electrodes of opposite charge, which is located epicardially between the superior vena cava and the aortic root, superior to the right pulmonary artery, the operational space including target tissue may be of maximum width dimension in the range of 4 to 8 cm, for example a spherical volume having a diameter of about 6 cm. The configuration may be such that the electrodes of said at least two electroporation electrodes which are oppositely charged have at least 2 mm of spacing therebetween. In embodiments, the electrodes of said at least two electroporation electrodes which are oppositely charged may have at least 5 mm of spacing therebetween.

The inferior aspect of the operational space may be positioned at least 2 mm above the transverse pericardial sinus, and optionally no more than 20 mm above the transverse pericardial sinus.

Considering a spherical volume of operational space, the vertical axis of such a "sphere" may be positioned midway between the nominally vertical axes of the superior vena cava and the ascending aorta.

### Example 2

### Apparatus for use in treating the aortic root ganglionated plexi

In an embodiment intended for treating the aortic root ganglionated plexi using at least two electroporation electrodes of opposite charge, the operational space including target tissue may be of maximum width dimension in the range of 4 to 8 cm, for example a spherical volume having a diameter of about 6 cm. The configuration may be such that the electrodes of said at least two electroporation electrodes which are oppositely charged have at least 2 mm of spacing therebetween. In embodiments, the electrodes of said at least two electroporation electrodes which are oppositely charged may have at least 10 mm of spacing therebetween.

Considering a spherical volume of operational space, the horizontal central axis of such a "sphere" can be aligned with the transverse sinus within a tolerance of +/-10mm. This places the operational space more inferior relative to the heart in comparison with the use in treating the aortocaval ganglionated plexi described in Example 1.

The vertical axis of the "sphere" may be positioned midway between the nominally vertical axes of the ascending aorta and the pulmonary trunk.

### Example 3

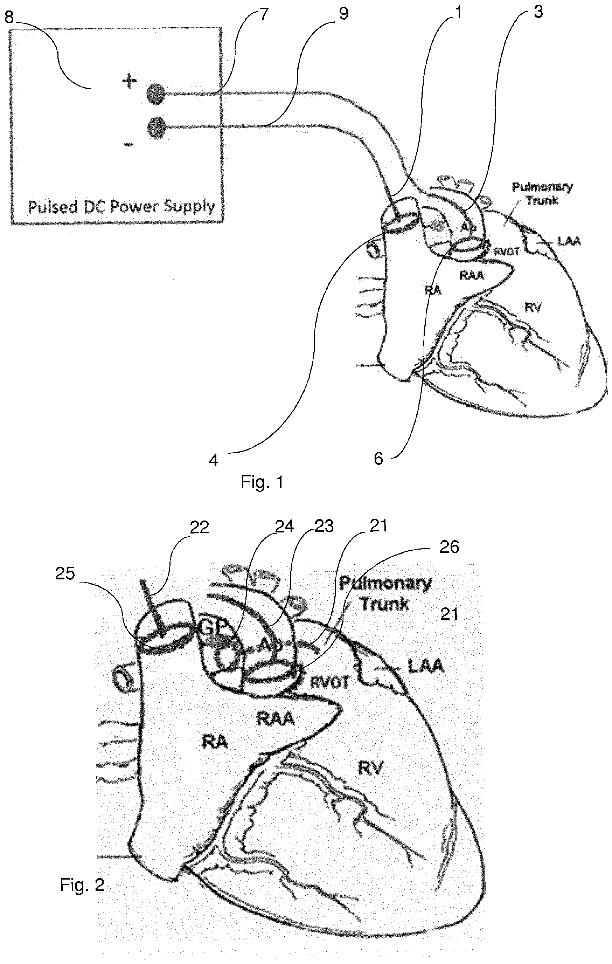

An apparatus suitable for carrying out an electroporation treatment (referring to **Fig. 1****)** comprises first and second catheters having respectively first and second catheter tips **(1; 3)** of the curved loop type, with each curved loop tip (1, 3) bearing multiple electrodes **(4; 6)** respectively connectable by means of electrical conductors **(7; 9)** to positive (+) and negative (-) poles of a pulsed direct current power supply **(8).**

In use one pole (-) of the pulsed direct current power supply (8) may be connected to a catheter tip (1) positioned in the superior vena cava, and the other pole (+) of the pulsed direct current power supply (8) is connected to a catheter tip located in the aorta (3). The pulsed direct current electric field will be at its peak strength in between these catheter tips. The choice of polarity of electrode at each catheter tip in contact with the tissue does not matter provided always that a potential difference is established and at least one electrode in contact with tissue is of an opposite charge from at least one other electrode in contact with tissue, for example, of at least two electrodes one is positive and the other is negative.

### Example 4

An apparatus connectable by means of electrical conductors to positive (+) and negative (-) poles of a pulsed direct current power supply, as in Example 3, and suitable for carrying out an electroporation treatment (referring to Fig. 2) comprises a catheter assembly including first, second and third catheter tips **(21; 22; 23)** of the curved loop type, each curved loop tip (21, 22, 23) bearing multiple electrodes **(24; 25; 26)** and being respectively positioned in the right pulmonary artery, superior vena cava, and aorta.

In use, with the three catheter tips in place, the electrical field can be applied between any selected two catheter tips coupled such that a slightly different field focus exists with each alternate couple but throughout the ganglionated plexi are continuously electroporated. An electroporation voltage of 1000 Volts may be applied in a pulse of approximately 100 microseconds.

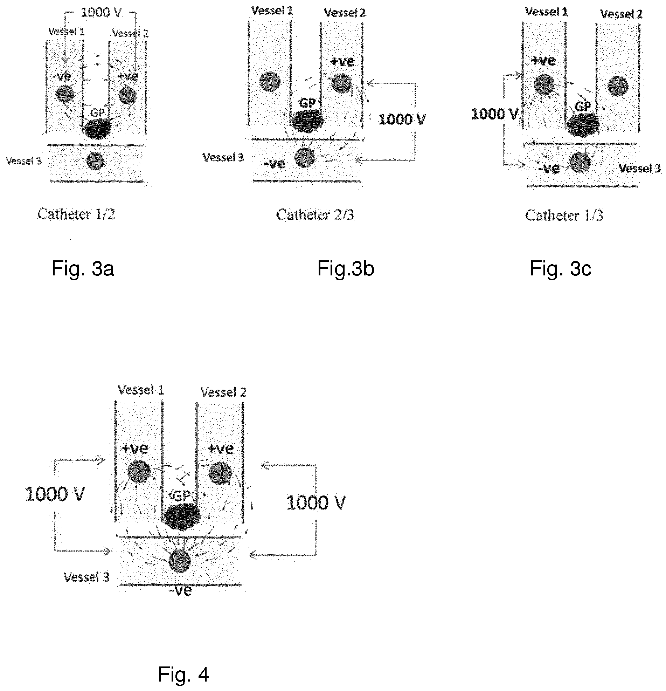

A possible sequence is illustrated in Figs. 3a, 3b, and 3c, wherein a first catheter tip is coupled with a second catheter tip; then after a period of electroporation, the second catheter tip is coupled with the third catheter tip; then after a period of electroporation the first catheter tip is coupled with the third catheter tip. The catheter tip/electrode assembly is schematically represented as a filled circle in a natural vessel close to the ganglionated plexi (GP) and the sequence of coupled charges applied is indicated by +ve and -ve for each stage. The electroporation period can be shorter than necessary to reach anticipated completion, and the said combinations may be cycled through in sequence with sufficient repetition to achieve completion.

### Example 5

The apparatus described for Example 4, may be used in a different method wherein two of the catheter tips are connected to the source to have the same polarity, whilst the third catheter tip is connected to the source to have the opposite polarity (as illustrated in Fig. 4. An electroporation voltage of 1000 Volts may be applied in a pulse of approximately 100 microseconds to provide higher field density focused on the ganglionated plexi.

### Example 6

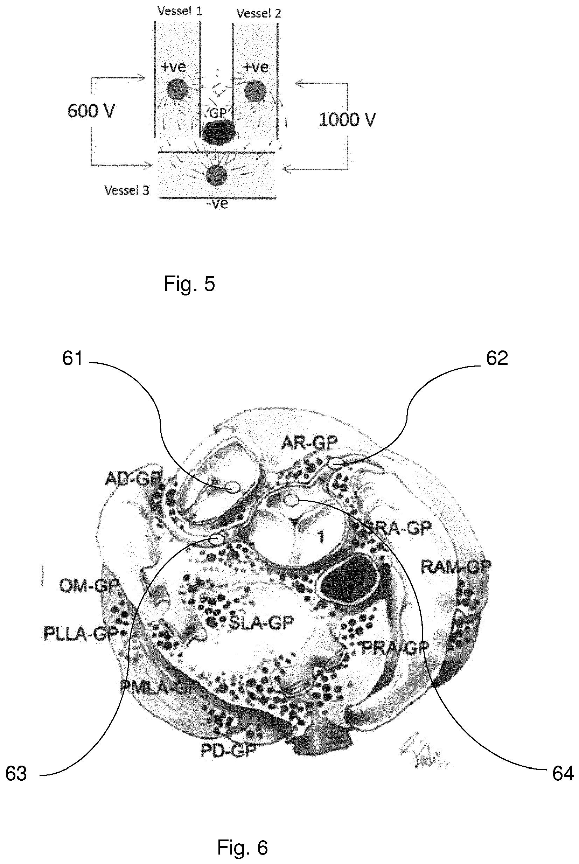

The apparatus described for Example 4, may be used in a different method wherein two of the catheter tips are connected to the source to have the same polarity, whilst the third catheter tip is connected to the source to have the opposite polarity. In this embodiment as illustrated in Fig. 5, the level of voltage applied differs between different electrode couples. For example, an enhanced field may be created by applying 600 Volts between the first and third catheter tips, and 1000 Volts between the second and third catheter tips, whereby the voltage difference between the first and third catheter tips means that a current and field will also exist between them.

It will be understood that any of the three catheter tip illustrative embodiments disclosed here can be selected for any arrangement within any of the pulmonary arteries, superior vena cava, and aorta in order to maximise the electrical field imparted to the aortocaval ganglia, i.e. creating different three-dimensional field geometries. The differing fields could be applied simultaneously, or pulsed in sequence between different paired catheter tip electrodes. Equally, the aforesaid embodiments may also apply to electroporation of the aortic root ganglionated plexi located on the upper surface of the left ventricle boundary, adjacent to the right coronary artery. This aortic root ganglionated plexi can be treated by inserting the catheter tip electrodes into combinations of the aorta **64** , pulmonary trunk **61,** right coronary artery **62** and left coronary artery **63.** In one illustrative procedure, a first catheter tip electrode may be positioned into the pulmonary trunk via the right atrium, right ventricle and just past the pulmonary valve. A second catheter tip electrode may be positioned into the proximal region of the right coronary artery, via the aorta, which permits creation of an electrical field between the first and second catheter tip electrodes to allow electroporation of the aortic root ganglionated plexi. Similarly, placing one catheter tip electrode in the aorta, just above the aortic valve, and a second catheter tip electrode 10-30 mm distally into the right coronary artery permits creation of another electrical field to allow electroporation of the aortic root ganglionated plexi. Alternatively placing catheter tip electrodes into the proximal regions of both left and right coronary arteries also allows electroporation of the aortic root ganglionated plexi (Fig. 6)..

In still further embodiments, using three catheter tip electrodes as described for Examples 4 to 6 above may also be utilised to create additional electrode field shapes, all intersecting and ablating the aortic root ganglionated plexi.

In any of the embodiments, a voltage of 1000 Volts may be needed, with an operational range for the apparatus of 500- 2000 Volts, being applied at a pulse duration of 100 microseconds, with an operational range of 1 microsecond to 1 millisecond. The resulting electroporation field strength will be in the region of 1000 Volts per centimetre depending on anatomical variations and positioning of the respective catheter tips. Multiple repeat pulses would be used, all pulses being monophasic. Such a region of field strength is anticipated to provide an operating zone that is primarily in the regime of reversible electroporation for myocardial tissue, but irreversible for ganglia structures in the fat pads..

### Example 7

A catheter tip and electrode assembly may be sheathed using a sheath having single or multiple apertures the size and shape of which may be the same or different.

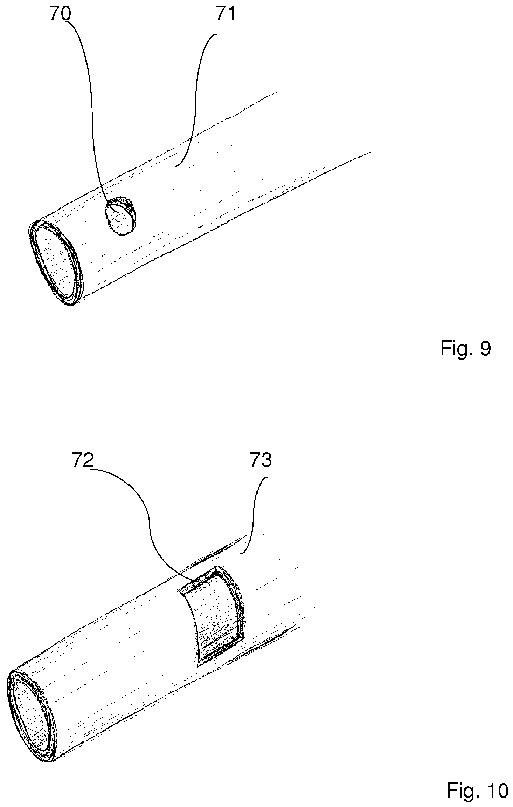

Referring to Fig. 9 a circular aperture **70** in a sheath **71** is illustrated, and the sheath 71 would be moveable upon a catheter (not shown) axially and rotationally with respect to the longitudinal axis of the catheter so as to be fully or partially aligned with an electrode on the catheter tip to adjust electrical field emission.

Referring to Fig. 10 a rectangular aperture **72** in a sheath **73** is illustrated, and the sheath 73 would be moveable upon a catheter (not shown) axially and rotationally with respect to the longitudinal axis of the catheter by a user so as to be fully or partially aligned with an electrode on the catheter tip to adjust electrical field emission.

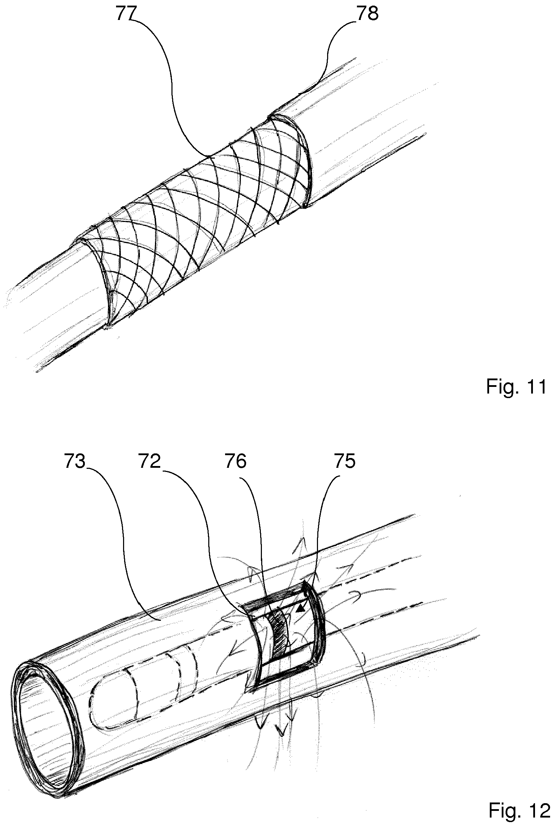

Referring to Fig. 12 a rectangular aperture 72 in a sheath 73 is illustrated, and the sheath is shown overlying a catheter tip and electrode assembly **75** such that the rectangular aperture or "window" is aligned with an electrode **76** on the catheter tip to allow electrical field emission.

The relative movement of the sheath with respect to the catheter can be achieved by a user manipulating a proximal handle of the catheter and/or a gripping portion of the overlying sheath.

The sheath may be a metal braided sheath as illustrated in Fig. 11 wherein a metal cellular pattern structure **77** serves as a Faraday cage concealed within a polymeric casing **78** of the sheath. The metal may be of any suitable conductive material such a conductive metal or alloy, for example copper, stainless steel or a nickel-titanium alloy (nitinol).

### Example 8

Various catheter tip and electrode assembly designs are useful for the purposes of the disclosed electroporation treatment. A suitable catheter has a throughbore through which electrical conductor wires can be passed to connect with the respective electrodes in the illustrated embodiments.

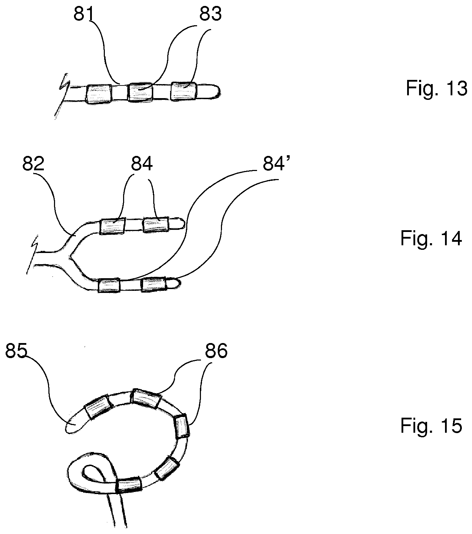

As illustrated in Fig. 13 a straight linear catheter tip **81** has spaced apart electrodes **83** attached to the catheter tip,

In Fig. 14 a bifurcated linear catheter tip **82** I shown which has spaced apart electrodes **84, 84'** on the respective limbs of the bifurcated catheter tip 82.

A loop type, curvilinear catheter tip **85** bearing spaced electrodes **86** is illustrated in Fig. 15.

Each of the electrodes can be selectively controlled to operate together or individually in a sequence and at varying electrical field output.

### Example 9

Referring to Figs/ 16 and 17, an electrode configured to fit over a catheter tip may be of a tubular form **90** with an irrigation hole **91** to allow through flow of a fluid. The fluid may be introduced through a hollow catheter introduced to a throughbore **100** of electrode of the tubular form 90, and having a corresponding aperture to register with the irrigation hole 91.

### Example 10

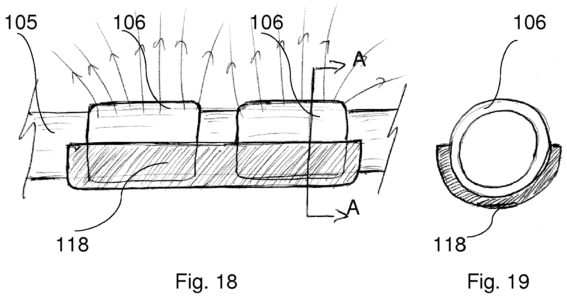

Fig. 18 illustrates schematically a portion of a catheter tip and electrode assembly which is partially insulated, the catheter **105** bearing two spaced electrodes **106** each partially covered by an insulator **118,** optionally the insulator 118 being a portion of an inflatable envelope or "balloon"; and Fig. 19 shows a cross-section [A-A] of the insulated electrode bearing potion of the catheter tip and electrode assembly

### Example 11

In a procedure requiring enhanced electroporation, targeting epicardial ganglionated plexi embedded in fat pads on the outside surface of the heart, it is considered that in order to preferentially target the ganglia and mitigate the electroporation effect on the underlying myocardium the effect of treatment temperature is to be taken account of. Effecting temperature control is achievable in the vicinity of the target ganglionated plexi by selective application of heat and/or cooling to tissue surfaces.

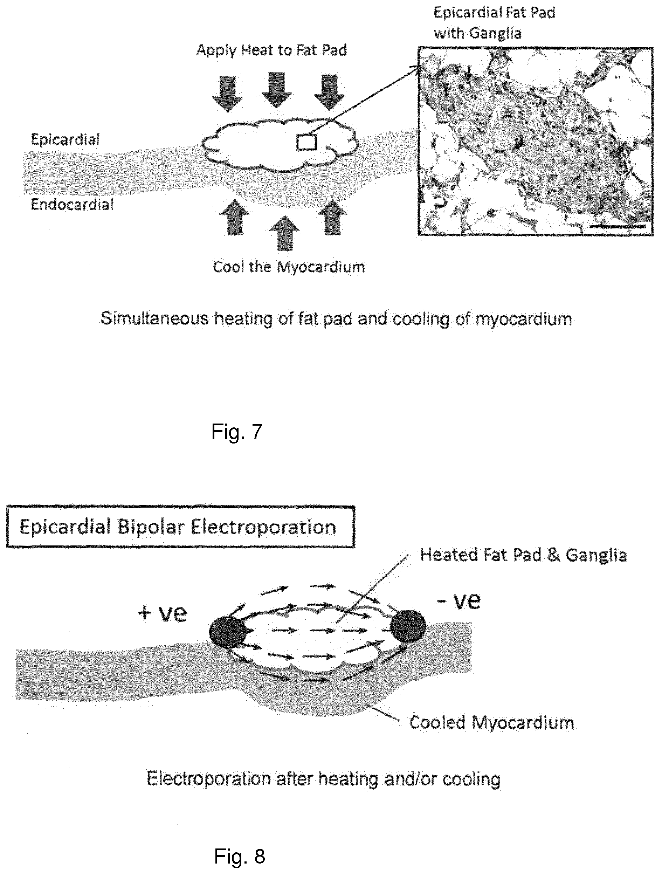

Referring to Fig. 7, an epicardial fat pad with ganglia is represented undergoing simultaneous temperature treatments such that by application of heat on the epicardial side, heating of the fat pad is achieved, and by application of cooling on the endocardial side, cooling of the myocardium is achieved. By selectively heating the fat pad the ganglia become more susceptible to electroporation and thus can be targeted at a lower electrical field strength, which mitigates the risk of unintended damage to the myocardium. This risk of unintended damage is still further managed by introducing cooling of the myocardium tissue surface.

Cooling of the myocardium is achievable by introducing a chilled saline balloon, whereby the temperature of the myocardium may be cooled locally to about 5-30 °C.

Heating of the fat pad is achievable by introducing a warm saline-containing balloon to overlie and contact the fat pad.

After the aforesaid temperature treatments are initiated, an electroporation apparatus including a bipolar catheter tip-electrode assembly may be introduced to contact epicardial tissue at spaced locations such that a (+) electrode of the catheter tip-electrode assembly is applied to the epicardial tissue or fat pad in the vicinity of the ganglia, and a (-) electrode is also applied to a different area of the epicardial tissue or fat pad in the vicinity of the ganglia (Fig. 8), the electrodes being connected to a D.C. source for effecting electroporation by means of an electrical field extending over the epicardial surface between the electrodes.

In alternative embodiments, an electrode, for example (+) electrode of the catheter tip-electrode assembly is introduced to contact epicardial tissue at a selected location of the epicardial tissue or fat pad in the vicinity of the ganglia, and another electrode of opposite polarity, in this example (-) electrode of the catheter tip-electrode assembly is introduced to contact myocardial tissue in the vicinity of the ganglia, the electrodes being connected to a D.C. source for effecting electroporation by means of an electrical field extending through the myocardium between the electrodes.

An electroporation apparatus suitable for carrying out the procedure including temperature treatments comprises an elongate tubular catheter having a proximal end and a distal end, and internal fluid channels. The distal end of the catheter has spaced electrodes on a side surface, and a recess between the electrodes. The recess houses an inflatable balloon in communication with the internal fluid channels for receiving and venting a temperature control fluid, such as heated or cooled saline which may be circulated under pressure. Electrical conductor wires located upon or within the elongate tubular catheter are connectable to an external direct current supply with controller to adjust the voltage of the electrical supply.

In an alternative embodiment, the inflatable balloon for receiving a temperature controlled fluid is housed in a distal end face recess, instead of in a side surface of the elongate tubular catheter.

In still further alternative embodiments, a unipolar electroporation apparatus has a single distal end electrode connected to a direct current electrical supply. When used in a unipolar manner a single electrode would be used in conjunction with a back pad/dispersive electrode of opposite polarity on the patient (sometimes called an "indifferent electrode").
Still further embodiments of the enhanced electroporation apparatus using differing combinations of electrodes, e.g. three electrodes as disclosed in Example 4 above are contemplated, and whilst examples are provided by way of illustration, it will be appreciated that these are indicative designs and that other designs with different overall shape and electrode arrangements are possible, so that these examples are non-limiting and attention is directed to the scope of the claims hereinafter appearing.

## Claims

1. Electrophysiology apparatus comprising a high voltage pulsed DC supply, and a catheter configured for endocardial access, the catheter including a catheter tip and electrode assembly connectable with the high voltage pulsed DC supply to provide an electroporation electrode.

2. The electrophysiology apparatus of claim 1, comprising at least two electroporation electrodes connectable with the high voltage pulsed DC supply such that the at least two electroporation electrodes are oppositely charged.

3. The electrophysiology apparatus of claim 1, wherein the catheter tip and electrode assembly comprises at least three electroporation electrodes connectable with the high voltage pulsed DC supply such that, selectively, at least two of the electroporation electrodes are oppositely charged.

4. The electrophysiology apparatus of claim 2 or claim 3, wherein the catheter tip and electrode assembly comprises a plurality of catheter tips and each catheter tip is provided with one electrode of the said at least two electroporation electrodes.

5. The electrophysiology apparatus of any one of the preceding claims wherein an electroporation electrode comprise a catheter tip bearing multiple electrodes operable individually, in selected combinations, or together at the same time.

6. The electrophysiology apparatus of any one of the preceding claims wherein an electroporation electrode is partially insulated to direct the electric field in a desired direction, and reduce the effect of the electric field in other directions.

7. The electrophysiology apparatus of any one of the preceding claims, comprising a sheath for electrical field blocking, wherein the sheath is configured to fit over the catheter tip and electrode assembly and to have a plurality of openings for selective electric field emission.

8. The electrophysiology apparatus of claim 7, wherein the sheath is adjustable circumferentially or longitudinally upon the catheter tip and electrode assembly to influence electrical field emission.

9. The electrophysiology apparatus of claim 7, or claim 8, wherein the sheath comprises a metal reinforced polymer.

10. The electrophysiology apparatus of any one of the preceding claims wherein the catheter tip and electrode assembly comprises an articulated catheter tip portion to allow the catheter tip to be deflected and re-oriented to alter the direction of an electrical field emanating therefrom.

11. The electrophysiology apparatus of any one of the preceding claims, wherein the electrodes of each catheter tip and electrode assembly are individually controllable by a controller operable by a user, or by a programmable control system incorporating a controller, wherein said controller selectively controls the pairing of (+) and (-) electrodes of the catheter tip and electrode assemblies according to a switching sequence, and controls the duration of an electrical field pulse delivered by the high voltage pulsed DC supply.

12. The electrophysiology apparatus of any one of the preceding claims, wherein the high voltage pulsed DC supply has an operable range of from 500 to 2000 Volts, and is controllable to deliver an electrical field pulse duration of from 1 microsecond to 1 millisecond.

13. The electrophysiology apparatus of any one of the preceding claims, wherein multiple catheters are provided, each catheter having a catheter tip and electrode assembly, whereby each catheter tip and electrode assembly is respectively insertable into a natural lumen or cavity adjacent to target tissue, and each catheter tip and electrode assembly is located in a different natural lumen or cavity adjacent to the target tissue, such that electrodes of respective catheter tip and electrode assemblies are positioned within the operational space of maximum width dimension in the range of 4 to 8 cm, and a (+) electrode is spaced from a coupling electrode by at least 2 mm.

14. The electrophysiology apparatus of any one of the preceding claims, wherein the catheter is configured to effect temperature treatments and comprises an elongate tubular body having a proximal end and a distal end, and internal fluid channels, the distal end of the catheter having spaced electrodes on a side surface, and a recess between the electrodes, wherein the recess houses an inflatable balloon in communication with the internal fluid channels for receiving and venting a temperature control fluid, such as heated or cooled saline circulated under pressure control for selective inflation of the inflatable balloon.

15. A system for carrying out electroporation of tissue comprising a high voltage pulsed DC supply, and at least one catheter configured for endocardial access, the catheter including a catheter tip and at least one electrode connectable with the high voltage pulsed DC supply for use with at least one separate electrode of opposite polarity, and a controller operably connected to the high voltage pulsed DC supply, and the electrodes for selectively controlling the electrodes according to a switching sequence, and controlling the duration of an electrical field pulse delivered by the high voltage pulsed DC supply.

16. A method of inhibiting atrial fibrillation of the heart by electroporation of ganglionated plexi, the method comprising introducing a catheter tip and electrode assembly endocardially via a lumen of a natural vessel selected from any of the superior vena cava, the aorta, pulmonary arteries, coronary arteries, to locate the catheter tip and electrode assembly adjacent to the ganglionated plexi, providing a high voltage pulsed direct current supply connectable to the catheter tip and electrode assembly to generate an electrical field, generating a pulsed electrical field around the catheter tip and electrode assembly adjacent to the ganglionated plexi, and applying repeated pulses of electrical field to effect electroporation of the ganglionated plexi.

17. The method of claim 16, wherein electroporation is effected in an operational space including ganglionated plexi by means of at least two electroporation electrodes connectable with the high voltage pulsed DC supply such that the at least two electroporation electrodes are oppositely charged, the said at least two electroporation electrodes having at least 2 mm of spacing therebetween, optionally at least 5 mm of spacing therebetween.

18. The method of claim 16 or claim 17, wherein the electroporation is conducted upon aortocaval ganglionated plexi and the at least two electroporation electrodes are positioned between the superior vena cava and the aortic root, superior to the right pulmonary artery.

19. The method of claim 18, the inferior aspect of the operational space being positioned at least 2 mm above the transverse pericardial sinus, and optionally no more than 20 mm above the transverse pericardial sinus.

20. The method of claim 16, wherein electroporation is effected in an operational space including ganglionated plexi by means of at least three electroporation electrodes connectable with the high voltage pulsed DC supply such that selectively at least two of the electroporation electrodes are oppositely charged, the said at least two electroporation electrodes having at least 2 mm of spacing therebetween, optionally at least 5 mm of spacing therebetween.

21. The method of any one of claims 16 to 20 wherein electrodes of the respective electroporation electrodes are selectively controllable to change at least one of applied voltage, pulse duration, coupling between electroporation electrodes, and charge polarity.

22. The method of any one of claims 16 to 21 wherein the catheter is manipulated to change electrical field direction by means of a fenestrated sheath movable axially and/ or rotationally upon the catheter.

23. The method of any one of claims 16 to 22 wherein the catheter is manipulated to change electrical field direction by means of the catheter tip and electrode assembly having a flexible portion or articulation point adjacent to the electrodes.
